# EUROPÄISCHE PATENTSCHRIFT

(11) **EP 0 519 329 B1**
(45) Veröffentlichungstag und Bekanntmachung des Hinweises auf die Patenterteilung: **15.01.1997**
(21) Anmeldenummer: 92109838.0
(22) Anmeldetag: 11.06.1992
(51) Int. Cl.: C07D 233/60, G03C 1/30

(54) **1,3-Bis-carbamoylimidazoliumverbindungen und Verfahren zum Härten von Gelatine enthaltenden Schichten**
1,3-Bis-carbamoyl imidazolium compounds and a process for hardening coatings containing gelatin
Composés d' 1-3-bis-carbamoylimidazolium et procédé pour durcir des couches contenant de la gélatine

(30) Priorität: 18.06.1991 DE 4119982
(43) Veröffentlichungstag der Anmeldung: 23.12.1992
(73) Patentinhaber: Sterling Diagnostic Imaging (Deutschland) GmbH, 61352 Bad Homburg (DE)
(72) Erfinder: Rüger, Reinhold, Dr., W-6074 Rödermark (DE)
(74) Vertreter: Lippert, Hans-Joachim, Dipl.-Ing.

(56) Entgegenhaltungen:
- EP-A- 0 344 468
- FR-A- 2 323 173
- GB-A- 2 247 237
- US-A- 4 751 173

## Beschreibung

Die Erfindung betrifft neue 1,3-Bis-carbamoylimidazoliumverbindungen sowie deren Verwendung in einem Verfahren zum Härten von Gelatine enthaltenden Schichten, insbesondere von Schichten photographischer Aufzeichnungsmaterialien.

Gelatine enthaltende Schichten werden auf verschiedenen Gebieten der Technik verwendet, beispielsweise als Schutzüberzüge von Gegenständen oder als Bindemittelschichten für Reagenzien in Materialien für analytische oder diagnostische Zwecke oder für lichtempfindliche Stoffe, vorzugsweise Silberhalogenide, in photographischen Aufzeichnungsmaterialien. Für den praktischen Gebrauch werden diese Schichten durch Zugabe eines Härtungsmittels gehärtet. Bekannte Härtungsmittel wirken durch Vernetzung der Gelatine infolge einer Reaktion mit deren freien Amino-, Imino- oder Hydroxylgruppen.

Dabei ist es erwünscht, daß die Härtungsreaktion innerhalb eines kurzen Zeitraums nach der Schichtbildung vollständig abläuft. Dadurch erhalten die Schichten sofort nach der Herstellung ihren vollen, auf der Härtung beruhenden Gebrauchswert. Insbesondere wird bei photographischen Aufzeichnungsmaterialien vermieden, daß sich die photographischen Eigenschaften noch während einer längeren Zeit nach der Herstellung ändern infolge sog. Nachhärtung ändern.

Sogenannte Soforthärter sind beispielsweise in den Veröffentlichungen EP-A2-02 57 515, DE-A1-38 19 082, DE-C2-24 39 551, DE-C2-23 17 677 und DE-C2-22 25 230 beschrieben. In diesen ist eine am Stickstoff zweifach substituierte Carbamoylgruppe an ein quaterniertes Stickstoffatom eines heterocyclischen Rings, im allgemeinen eines Pyridinrings, gebunden.

Die bekannten Soforthärter besitzen jedoch nur eine geringe Stabilität in wäßriger Lösung. Es ist daher nicht möglich, aus diesen Verbindungen wäßrige Vorratslösungen für die praktische Verwendung herzustellen, da die Härtungswirkung als Folge des mit der Lagerung abnehmenden Gehalts an Wirkstoff mit der Zeit geringer wird. Außerdem zeigen mit Pyridiniumhärtern hergestellte Lösungen und Schichten beim Gebrauch einen unangenehmen Pyridingeruch.

Aufgabe der Erfindung ist es daher, Soforthärter bereitzustellen, die in wäßriger Lösung stabil sind und im Gebrauch geruchfreie gehärtete Schichten ergeben.

Diese Aufgabe wird gelöst durch 1,3-Bis-carbamoylimidazoliumverbindungen der allgemeinen Formel 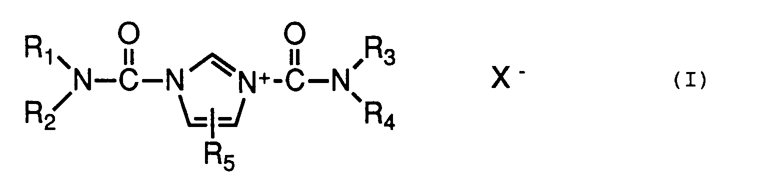 Hierin können R₁, R₂, R₃ und R₄ gleich oder verschieden sein und bedeuten je eine Alkylgruppe mit 1, 2 oder 3 Kohlenstoffatomen oder eine Phenyl- oder Benzylgruppe, auch können sowohl R₁ und R₂ als auch R₃ und R₄ zu einem fünf- oder sechsgliedrigen gesättigten Ring, der ein weiteres, mit Methyl, Ethyl oder Propyl substituiertes Stickstoffatom oder ein Sauerstoffatom enthalten kann, verbunden sein,
bedeutet R₅ Wasserstoff oder eine an einem der Kohlenstoffatome des Imidazolrings substituierende Alkylgruppe mit 1 bis 3 Kohlenstoffatomen, d.h. eine Methyl-, Ethyl-, Propyl- oder Isopropylgruppe
und bedeutet X⁻ ein Anion.

Der gegebenfalls von R₁ und R₂ bzw. R₃ und R₄ unter Beteiligung des Stickstoffatoms der Carbamoylgruppe gebildete fünf- oder sechsgliedrige Ring kann zum Beispiel ein Oxazolidin-, 3-Methylimidazolidin-, Morpholin-, Piperidin-, 4-Methylpiperazin- oder 3-Methyltetrahydropyrimidinring sein.

Das Anion X⁻ ist beispielsweise ein Chlorid-, Perchlorat-, Tetrafluoroborat- oder Toluolsulfonatanion.

Beispiele für erfindungsgemäße Substanzen sind

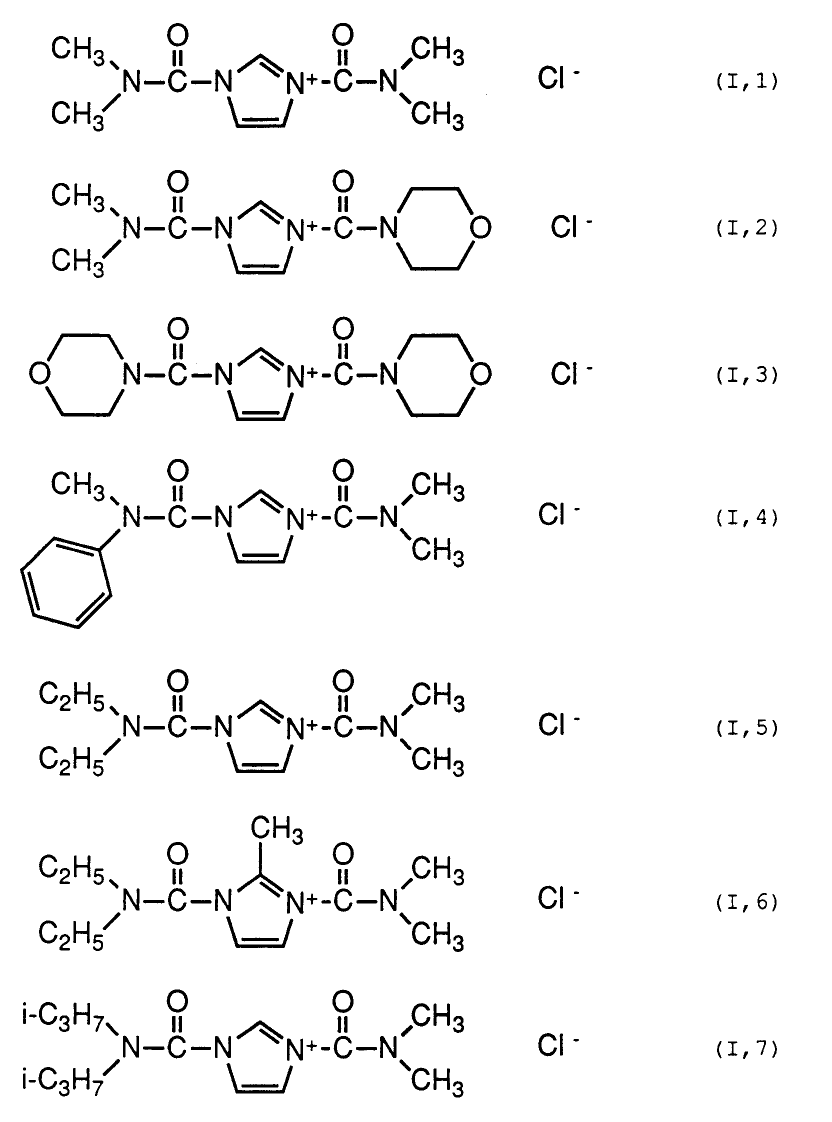

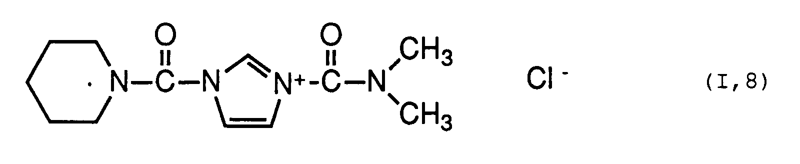

Die erfindungsgemäßen Verbindungen sind auf verschiedenen Synthesewegen zugänglich. Zur Herstellung einer bestimmten Verbindung kann daher ein optimaler Weg ausgewählt werden.

Die Synthese geht in der Regel vom Imidazolkern aus und verläuft in zwei Stufen, wobei das Imidazol zunächst mit einem Äquivalent eines Carbamoylchlorids und das resultierende Zwischenprodukt danach mit einem weiteren Äquivalent des gleichen oder eines anderen Carbamoylchlorids umgesetzt wird. So kann man sowohl symmetrisch als auch unsymmetrisch aufgebaute Verbindungen erhalten.

Beispielsweise kann man Imidazol in Gegenwart eines Säurefängers, zum Beispiel Triethylamin, mit einem Äquivalent eines N,N-Dialkylcarbamoylchlorids umsetzen. Bevorzugte Lösungsmittel sind hierbei Aceton und Tetrahydrofuran. Das salzartige Triethylammoniumchlorid fällt dabei aus und das Zwischenprodukt kann in der abgetrennten Lösung mit einem weiteren Äquivalent eines Dialkylcarbamoylchlorids zur Bis-carbamoylverbindung umgesetzt werden. Diese kristallisiert in für die Verwendung hinreichend reiner Form und kann leicht abfiltriert werden.

Das Zwischenprodukt kann auch durch Umsetzung von Imidazolnatrium mit einem Äquivalent eines Carbamoylchlorids in einem polaren Lösungsmittel, beispielsweise Tetrahydrofuran, und Abtrennung des ausfallenden Natriumchlorids gewonnen und wie oben in der Lösung weiter zur Bis-carbamoylverbindung verarbeitet werden.

Eine weitere Möglichkeit für die Herstellung der Monocarbamoylimidazol-Zwischenprodukte besteht in der Umsetzung von Carbonyldiimidazol mit einem Äquivalent eines sekundären Amins. Vor der weiteren Umsetzung muß man das Zwischenprodukt isolieren und umkristallisieren, um das in der ersten Stufe gebildete Imidazol zu entfernen.

Die 1,3-Bis-carbamoylimidazoliumverbindungen können sowohl allein als auch im Gemisch mit anderen bekannten Härtungsmitteln, beispielsweise Triazinverbindungen oder 2,3-Dihydroxy-1,4-dioxan, angewendet werden.

Die anzuwendende Menge des Härtungsmittels richtet sich nach der gewünschten Wirkung. Für die Härtung photographischer Schichten ist der Bereich von 0,02 bis 0,3 millimol je g Gelatine bevorzugt.

Das Verfahren zur Härtung von gelatinehaltigen Schichten wird bevorzugt so durchgeführt, daß man eine wäßrige gelatinehaltige Beschichtungslösung vor dem Auftragen mit einer wäßrigen Lösung des Härtungsmittels vermischt.

Besonders bevorzugt ist es, dieses Vermischen unmittelbar vor dem Auftragen durchzuführen, beispielsweise indem man die Lösung des Härtungsmittels in den Strom der Beschichtungslösung nahe der Beschichtungsvorrichtung injiziert.

Bevorzugt angewendet wird das erfindungsgemäße Verfahren auf gelatinehaltige Schichten, die Bestandteile photographischer Aufzeichnungsmaterialien sind. Dies können lichtempfindliche Silberhalogenid-Emulsionsschichten sein, die beispielsweise unsensibilisierte Emulsionen, orthochromatische, panchromatische oder infrarotempfindliche Emulsionen, farbkupplerhaltige Emulsionen, Röntgenemulsionen oder ultrasteile Emulsionen für die Reproduktion enthalten, aber auch Schutzschichten, Filterschichten, Antihalo- oder Anticurlschichten oder ganz allgemein photographische Hilfsschichten. In der Regel enthalten photographische Aufzeichnungsmaterialien, insbesondere solche für die Farbphotographie, mehrere solche Schichten.

Zur Härtung mehrerer auf einer Seite des Schichtträgers übereinander angeordneter Schichten photographischer Aufzeichnungmaterialien wird das Härtemittel bevorzugt nur einer Schicht, beispielsweise einer Unter- oder Übergußschicht, zugesetzt, die selbst auch frei von Gelatine sein kann. Dies kann auch eine Hilfsschicht, zum Beispiel in einem Verfahren nach der EP-A2-02 57 515, sein. Es ist auch möglich, eine härtemittelhaltige Lösung in einem besonderen Verfahrensschritt nachträglich auf die gelatinehaltigen Schichten aufzutragen.

Eine andere bevorzugte Verwendung finden die erfindungsgemäßen Verbindungen als Peptidkupplungsreagenzien, zum Beispiel bei der Synthese von Polypeptiden oder zur Verankerung von Polypeptiden wie Gelatine auf Oberflächen oder an löslichen Polymeren, welche Carbonsäurefunktionen aufweisen.

Die 1,3-Bis-carbamoylimidazoliumverbindungen sind auf einfache Weise und aus leicht zugänglichen Ausgangsstoffen herstellbar. Sie härten ebenso rasch wie bekannte Soforthärter, sind aber im festen Zustand und in wäßriger Lösung wesentlich beständiger. Die Stoffe selbst sowie ihre Lösungen und die mit ihnen gehärteten gelatinehaltigen Schichten sind frei von lästigen und schädlichen Gerüchen.

Die nach dem erfindungsgemäßen Verfahren gehärteten Schichten zeigen keine Nachhärtung. Bei photographischen Aufzeichnungsmaterialien erreichen daher die photographischen Eigenschaften, insbesondere die Empfindlichkeit und die Maximaldichte, bereits kurze Zeit nach dem Beschichten und Trocknen ihre Endwerte und ändern sich bei längerer Lagerung nicht mehr.

Die Erfindung kann zum Härten von gelatinehaltigen Schichten aller Art, beispielsweise bei der Herstellung von Schutzüberzügen auf Gegenständen, von Schichten für analytische oder diagnostische Zwecke, die reaktive Stoffe enthalten, insbesondere aber von lichtempfindlichen Schichten und Hilfsschichten in photographischen Aufzeichnungsmaterialien, angewendet werden.

### Ausführungsbeispiele:

### Beispiel 1

### Herstellung von 1,3-Bis-(dimethylcarbamoyl-)imidazoliumchlorid (Verbindung I-1)

Zu einer Lösung von 6,8 g (0,1 mol) Imidazol und 11 g Triethylamin in 70 ml trockenem Aceton werden unter Rühren 10,75 g (0,1 mol) Dimethylcarbamoylchlorid tropfenweise zugegeben. Unter Erwärmung fällt ein weißer Niederschlag von Triethylammoniumchlorid aus. Nach 5 Stunden Stehenlassen bei Raumtemperatur wird filtriert und dem Filtrat nochmals 10,75 g Dimethylcarbamoylchlorid zugesetzt. Nach 2 Tagen bei Raumtemperatur wird das kristalline Produkt abfiltriert, mit Aceton gewaschen und im Vakuum getrocknet. Ausbeute 20,2 g 1,3-Bis-(dimethylcarbamoyl-)imidazoliumchlorid (77 % der Theorie), Schmelzpunkt 115 °C.

| | | | | |
|---|---|---|---|---|
| Analyse: | Berechnet | C 43,8 % | H 6,08 % | N 22,7 % |
| | Gefunden | C 43,7 % | H 6,2 % | N 22,8 %. |

### Beispiel 2

### Herstellung von 1,3-Bis-(morpholinocarbonyl-)imidazoliumchlorid (Verbindung I-3)

Zu einer Lösung von 3,4 g (0,05 mol) Imidazol und 5,5 g Triethylamin in 60 ml trockenem Tetrahydrofuran werden 7,5 g (0,05 mol) Morpholino-4-carbonylchlorid gegeben. Die Mischung wird 30 min bei 50 °C gerührt; danach wird vom ausgefallenen Triethylammoniumchlorid abfiltriert. Dem Filtrat werden weitere 7,5 g Morpholino-4-carbonylchlorid zugesetzt. Nach zweitägigem Stehen bei Raumtemperatur haben sich Kristalle von 1,3-Bis-(morpholinocarbonyl-)imidazoliumchlorid ausgeschieden, die abfiltriert, mit Ether gewaschen und im Vakuum getrocknet werden. Ausbeute 9,6 g (58 % der Theorie), Schmelzpunkt 116 °C.

| | | | | |
|---|---|---|---|---|
| Analyse: | Berechnet | C 48,1 % | H 6,9 % | N 20,4 % |
| | Gefunden | C 48,0 % | H 7,0 % | N 20,6 %. |

In den nachstehenden Anwendungsbeispielen wurden die folgenden Vergleichsverbindungen eingesetzt:
- V-1:: Dimethylcarbamoylpyridiniumchlorid
- V-2:: Morpholinocarbonylpyridiniumchlorid
- V-3:: 1-Dimethylcarbamoyl-3-methylimidazoliumchlorid
- V-4:: 1-Morpholinocarbonyl-(pyridinium-4-ethansulfonat).NaCl.

### Beispiel 3

Zu einer fünfprozentigen wäßrigen Lösung einer photographischen Gelatine wurden ein dispergiertes Kupferphthalocyaninpigment in einer Menge von 1 Gewichtsprozent (bezogen auf die Gelatine) und ein anionisches Netzmittel zugefügt. Teile dieser Lösungen wurden mit den in der Tabelle 1 angegebenen Härtemitteln (jeweils 0,1 millimol je g Gelatine) in Form frisch bereiteter wäßriger Lösungen mit etwa 5 Gewichtsprozent versetzt und mit einer Rakel auf Polyethylenterephthalatfolie zu Schichten von etwa 60 µm Dicke verteilt. Nach einstündigem Trocknen bei Raumtemperatur wurden Proben der so hergestellten Filme in Wasser von 60 °C getaucht und auf Geruch geprüft. Weitere Proben wurden in 0,5 n Natronlauge von 25 °C eingehängt. Die Lauge wurde mit 1 K/min erwärmt und der Beginn des Abschmelzens der Gelatineschicht beobachtet. Diese Prüfung wurde nach einer Lagerzeit der Filme von 2 und 10 Tagen wiederholt.

**Tabelle 1**

| Härtemittel | Abschmelzbeginn (°C) nach Lagerzeit | | | Geruch |
|---|---|---|---|---|
| | 1 Stunde | 2 Tage | 10 Tage | |
| I-1 | 50 | 55 | 55 | keiner |
| I-2 | 48 | 55 | 55 | keiner |
| I-3 | 44 | 50 | 51 | keiner |
| I-5 | 50 | 55 | 56 | keiner |
| I-6 | 38 | 54 | 58 | keiner |
| V-1 | 56 | 60 | 61 | Pyridin |
| V-2 | 50 | 56 | 58 | Pyridin |
| V-3 | 29 | 43 | 50 | keiner |

Die Ergebnisse zeigen, daß sowohl die erfindungsgemäßen Verbindungen als auch die Vergleichsverbindungen V-1 und V-2 als Soforthärter wirken, d.h. bereits nach einer Stunde ist der Abschmelzbeginn bis nahe an den Endwert angestiegen und nach 2 Tagen ist die Härtung fast völlig abgeschlossen. Dagegen ist V-3 bei dieser Anwendungsweise kein Soforthärter.

### Beispiel 4

Auf einen Schichtträger wurden eine Gelatine-Silberhalogenid-Emulsion und darüber eine Gelatinelösung als Überguß gleichzeitig aufgetragen. Die in Emulsion und Überguß enthaltenen Gelatinemengen verhielten sich wie 4:1. Zu der Übergußlösung wurde 5 min vor dem Auftragen eine wäßrige Lösung eines in der Tabelle 2 angegeben Härtemittels zugegeben. Die Härtemittelmenge betrug stets 0,1 millimol je g der in Emulsion und Überguß enthaltenen gesamten Gelatinemenge. Der Versuch wurde sowohl mit einer frisch bereiteten Lösung des Härtemittels als auch mit einer 2 Tage bei Raumtemperatur gelagerten Lösung durchgeführt.

Eine Stunde nach dem Auftragen wurden die Filme in einem Entwickler der Zusammensetzung

| | |
|---|---|
| Wasser | 600 ml |
| Kaliumhydroxid | 30 g |
| Kaliumdisulfit | 66 g |
| EDTA | 3 g |
| Kaliumbromid | 3 g |
| Benzotriazol | 0,5 g |
| Phenylmercaptotetrazol | 0,05 g |
| Hydrochinon | 25 g |
| N-Methyl-p-aminophenolsulfat | 1,5 g |
| Natriumcarbonatmonohydrat | 48 g |
| Diethylaminopropandiol | 25 g |
| pH eingestellt auf | 10,9 bei 20 °C |
| Wasser | zu 1 l |

und in einem handelsüblichen Fixierbad mit einem Gehalt an einem Aluminiumsalz mittels einer handelsüblichen Entwicklungsmaschine bei 36 °C und einer Entwicklungszeit von 40 s verarbeitet. Beurteilt wurde die Maschinengängigkeit der Versuchsfilme:
Ja, wenn der ausfixierte und getrocknete Film klar war und eine glatte glänzende Oberfläche besaß;
nein, wenn der Film trüb mit matter Oberfläche oder die Emulsionsschicht ganz oder teilweise abgelöst war.

**Tabelle 2**

| Probe Nr. | Härter Maschinengängigkeit | Alter der Härterlösung | |
|---|---|---|---|
| 1 | I-1 | frisch | ja |
| 2 | I-1 | 2 Tage | ja |
| 3 | V-1 | frisch | ja |
| 4 | V-1 | 2 Tage | ja |
| 5 | V-2 | frisch | ja |
| 6 | V-2 | 2 Tage | nein |
| 7 | V-3 | frisch | nein |
| 8 | V-3 | 2 Tage | nein |
| 9 | V-4 | frisch | ja |
| 10 | V-4 | 2 Tage | nein |

Diese Ergebnisse zeigen, daß die gealterten Lösungen der Vergleichssubstanzen V-2 und V-4 keine ausreichende Härtungswirkung besitzen, um Machinengängigkeit der Filme zu gewährleisten. V-3 wirkt erwartungsgemäß nicht als Soforthärter. Demgegenüber ist die gealterte Lösung der erfindungsgemäßen Substanz I-1 wenigstens ebenso gut wie die beste der Vergleichssubstanzen, V-1.

### Beispiel 5

Es wurden jeweils 0,25 molare wäßrige Lösungen der in Tabelle 3 angegebenen Härtemittel hergestellt und der Gehalt an Wirksubstanz nach verschieden langer Lagerung der Lösungen bei 20 °C durch UV Photometrie an verdünnten Proben bestimmt. Die Ergebnisse zeigen die überlegene Stabilität der erfindungsgemäßen Verbindung I-1 selbst gegenüber der stabilsten Vergleichssubstanz V-1.

**Tabelle 3**

| Härtungsmittel | Gehalt der Lösung in Prozent des Anfangswertes nach | | | |
|---|---|---|---|---|
| | 0 Stunden | 10 Stunden | 2 Tagen | 7 Tagen |
| I-1 | 100 | 100 | 100 | 100 |
| V-1 | 100 | 95 | 88 | 60 |
| V-2 | 100 | 50 | <10 | 0 |

## Patentansprüche

1. 1,3-Bis-carbamoylimidazoliumverbindungen der allgemeinen Formel 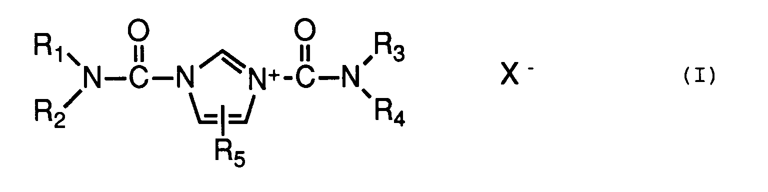 hierin
können R₁, R₂, R₃ und R₄ gleich oder verschieden sein und bedeuten je eine Alkylgruppe mit 1, 2 oder 3 Kohlenstoffatomen oder eine Phenyl- oder Benzylgruppe,
auch können sowohl R₁ und R₂ als auch R₃ und R₄ zu einem fünf- oder sechsgliedrigen gesättigten Ring, der ein weiteres, mit Methyl, Ethyl oder Propyl substituiertes Stickstoffatom oder ein Sauerstoffatom enthalten kann, verbunden sein,
bedeutet R₅ Wasserstoff oder eine an einem der Kohlenstoffatome des Imidazolrings substituierende Alkylgruppe mit 1 bis 3 Kohlenstoffatomen
und bedeutet X⁻ ein Anion.

2. Verfahren zum Härten von Gelatine enthaltenden Schichten,
dadurch gekennzeichnet, daß
als Härtemittel eine 1,3-Bis-carbamoylimidazoliumverbindung nach Anspruch 1 verwendet wird.

3. Verfahren nach Anspruch 2,
dadurch gekennzeichnet, daß
als Härtemittel eine der folgenden Verbindungen verwendet wird:

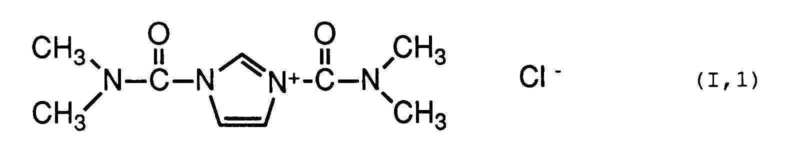

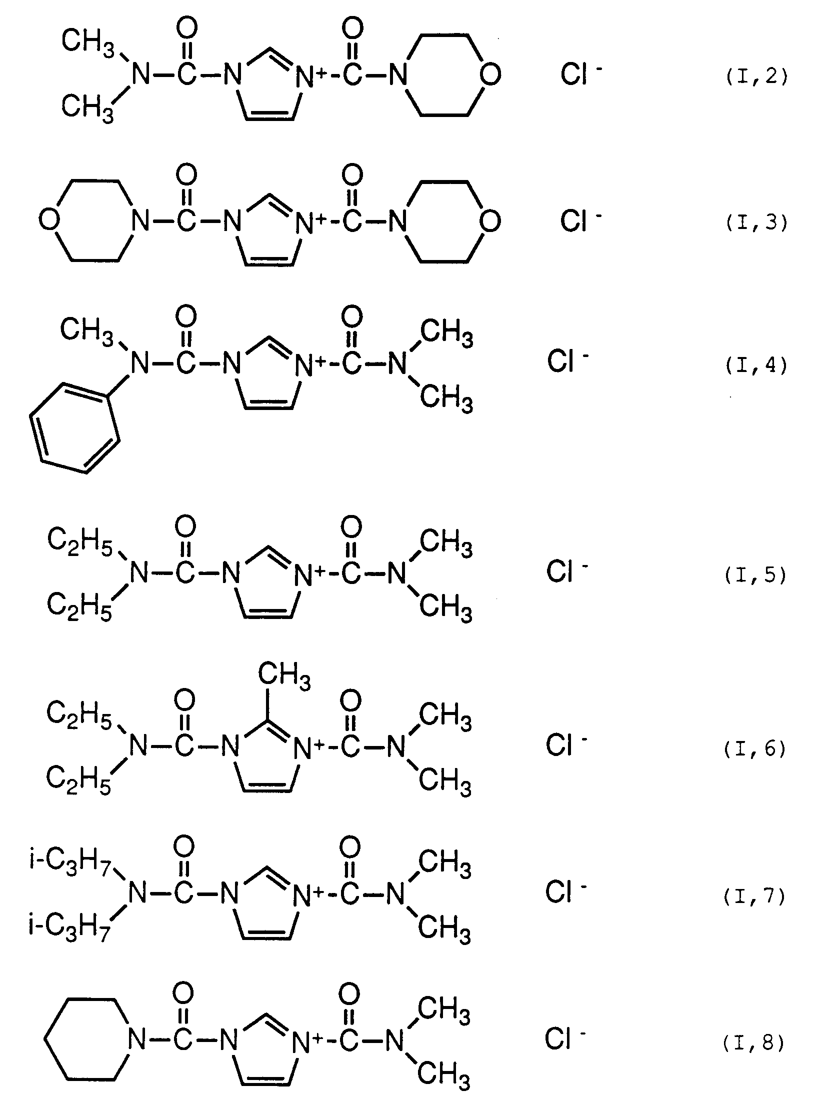

4. Verfahren nach Anspruch 2 oder 3,
dadurch gekennzeichnet, daß
das Härtemittel den Beschichtungslösungen unmittelbar vor dem Auftragen auf den Schichtträger zugesetzt wird.

5. Verfahren nach einem der Ansprüche 2 bis 4,
dadurch gekennzeichnet, daß
die Schichten Bestandteile photographischer Aufzeichnungsmaterialien sind.

6. Verfahren nach Anspruch 5,
dadurch gekennzeichet, daß
das Härtemittel der Beschichtungslösung für nur eine von mehreren Schichten zugesetzt wird.

7. Photographisches Aufzeichnungsmaterial mit mindestens einer gelatinehaltigen Schicht,
dadurch gekennzeichnet, daß
die gelatinehaltige Schicht mittels eines Verfahrens nach einem der Ansprüche 2 bis 6 gehärtet ist.

8. Verwendung der 1,3-Bis-carbamoylimidazoliumverbindungen nach Anspruch 1 als Peptidkupplungsreagenz.

## Claims

1. 1,3-Bis-carbamoylimidazolium compounds of general formula (I) 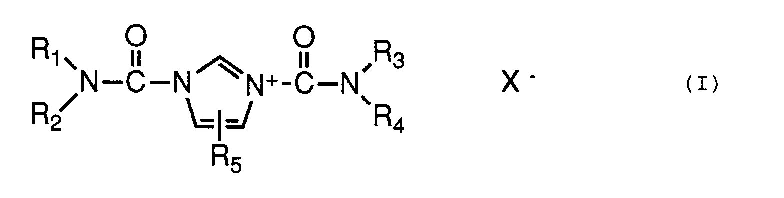 wherein
R₁, R₂, R₃ and R₄ may be equal or different and represent each an alkyl group with 1, 2, or 3 carbon atoms or a phenyl or a benzyl group,
R₁ und R₂ as well as R₃ und R₄ can be linked to form a five or six membered saturated ring which may comprise another nitrogen atom substituted with methyl, ethyl, or propyl, or an oxygen atom,
R₅ represents hydrogen or an alkyl group substituting at a carbon atom of the imidazole ring and having 1 to 3 carbon atoms, and
X⁻ represents an anion.

2. A method for hardening of coatings containing gelatin,
characterized in that
a 1,3-bis-carbamoylimidazolium compound according to claim 1 is utilized as a hardener.

3. The method of claim 2,
characterized in that
one of the following compounds is used as a hardener

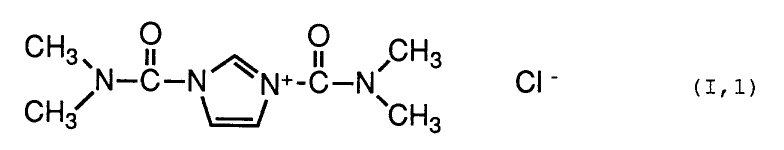

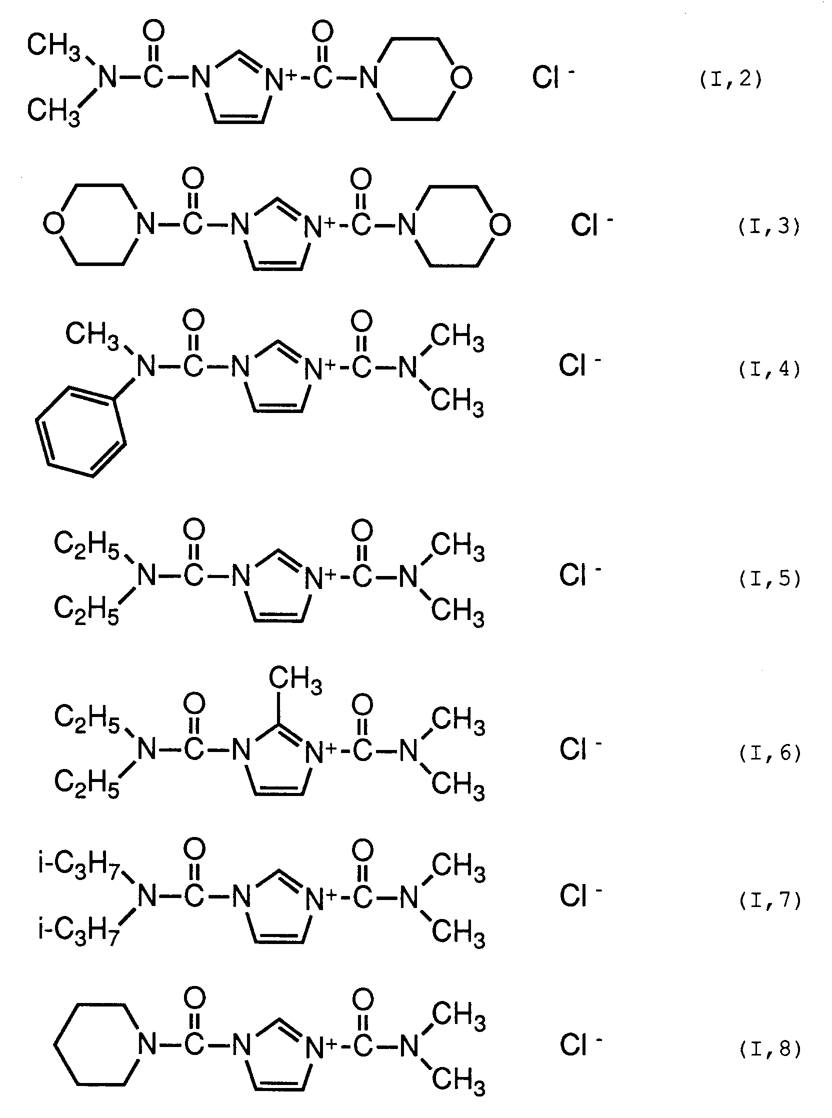

4. The method of claim 2 or claim 3,
characterized in that
the hardener is added to the coating solutions immediately before being coated on a support.

5. The method of on of claims 2 to 4,
characterized in that
the coatings are components of photographic recording materials.

6. The method of claim 5,
characterized in that
the hardener is added to the coating solution of just one of a plurality of coatings.

7. A photographic recording material comprising at least one gelatin containing coating,
characterized in that
the gelatin containing coating was hardened by a method according to one of claims 2 to 6.

8. Use of the 1,3-bis-carbamoylimidazolium compounds according to claim 1 as peptide coupling reagent.

## Revendications

1. Composés 1,3-bis-carbamoylimidazolium de formule générale 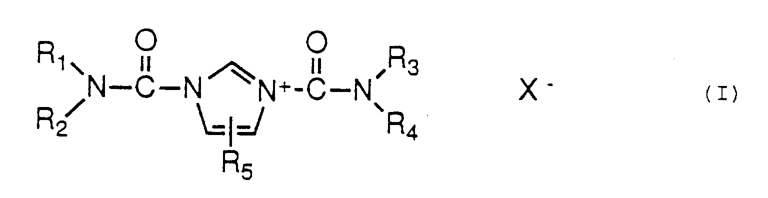 dans laquelle
R₁, R₂, R₃ et R₄ peuvent être identiques ou différents et représentent chacun un groupement alkyle à 1, 2 ou 3 atomes de carbone ou un groupement phényle ou benzyle, R₁ et R₂, ou R₃ et R₄, pouvant également être reliés pour former un cycle saturé à 5 ou 6 maillons, qui peut contenir un atome d'azote supplémentaire substitué par un groupement méthyle, éthyle ou propyle, ou un atome d'oxygène,
R₅ représente un atome d'hydrogène ou un groupement alkyle ayant 1 à 3 atomes de carbone et venant substituer l'un des atomes de carbone du cycle imidazolique,
et X⁻ représente un anion.

2. Procédé de durcissement de couches contenant de la gélatine, caractérisé en ce que l'on utilise comme agent durcissant un composé 1,3-bis-carbamoylimidazolium selon la revendication 1.

3. Procédé selon la revendication 2, caractérisé en ce que l'on utilise comme agent durcissant un des composés suivants :

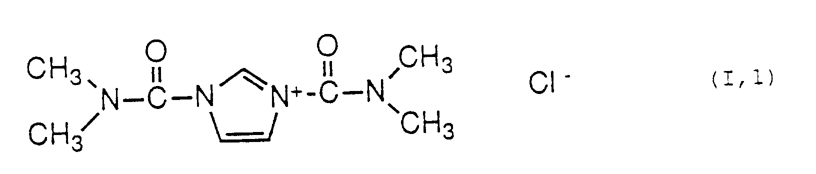

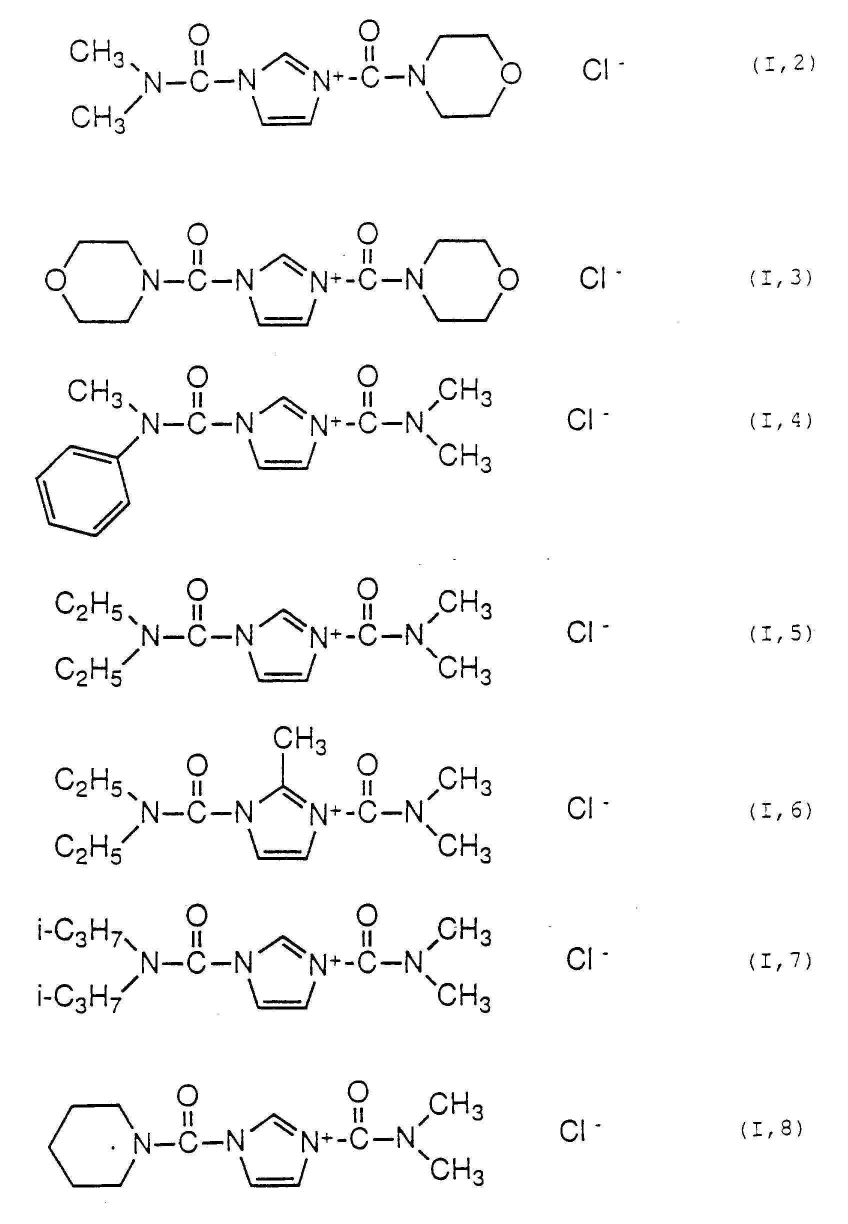

4. Procédé selon la revendication 2 ou 3, caractérisé en ce que l'agent durcissant est ajouté aux solutions de revêtement directement avant le dépôt sur le support de la couche.

5. Procédé selon l'une quelconque des revendications 2 à 4, caractérisé en ce que les couches sont des constituants de matériaux d'enregistrement photographiques.

6. Procédé selon la revendication 5, caractérisé en ce que l'agent durcissant n'est ajouté qu'à la solution de revêtement destinée à une couche parmi plusieurs.

7. Matériel d'enregistrement photographique avec au moins une couche contenant de la gélatine, caractérisé en ce que la couche contenant de la gélatine est durcie au moyen d'un procédé selon l'une quelconque des revendications 2 à 6.

8. Utilisation de composés 1,3-bis-carbamoylimidazolium selon la revendication 1 en tant que réactifs de copulation de peptides.
